# EUROPEAN PATENT APPLICATION

(11) **EP 4 752 159 A1**
(43) Date of publication of application: **03.06.2026**
(21) Application number: 25219600.1
(22) Date of filing: 29.11.2025
(51) Int. Cl.: C07K 16/26, G01N 33/74

(54) **ANTIBODY AGAINST ESTRADIOL IMMUNOCOMPLEX AND KIT THEREOF**

(30) Priority: 29.11.2024 CN 202411749558
(71) Applicant: Shenzhen Mindray Bio-Medical Electronics Co., Ltd., Shenzhen, Guangdong 518057 (CN); Hytest Ltd., 20520 Turku (FI)
(72) Inventor: FILATOV, Vladimir L., 117186 Moscow (RU); POPOVA, Anfisa S., 117186 Moscow (RU); KOKISHEVA, Antonina S., 117186 Moscow (RU); ZHUKOVA, Zlata A., 117186 Moscow (RU); SOKOLOVA, Elizaveta E., 117186 Moscow (RU); KATRUKHA, Alexey G., 117186 Moscow (RU); KOZLOVSKY, Stanislav V., 117186 Moscow (RU); POSTNIKOV, Alexander B., 117186 Moscow (RU); JIANG, Lei, Shenzhen, 518057 (CN); ZHAN, Chengxiong, Shenzhen, 518057 (CN); LIU, Kun, Shenzhen, 518057 (CN); CHEN, Puguang, Shenzhen, 518057 (CN); TANG, Tao, Shenzhen, 518057 (CN)
(74) Representative: KIPA AB

(57) **Abstract**

The present invention belongs to the fields of medicine and detection, and relates to an antibody against estradiol immunocomplex and kit thereof. The antibody of the present invention is capable of specifically binding to estradiol immunocomplex, but incapable of binding to estradiol alone or to estradiol-specific antibody alone. A kit prepared using the antibody of the present invention is capable of effectively measuring estradiol with extremely high sensitivity and specificity.

## Description

### Technical Field

The present invention belongs to the fields of medicine and detection, and relates to an antibody against estradiol immunocomplex and kit thereof. The estradiol immunocomplex refers to a complex formed by binding estradiol to an anti-estradiol antibody. Specifically, the kit is a kit for detecting or measuring estradiol level.

### Background

Estradiol (E2) is a type of estrogen that regulates multiple functions in the human body. It controls ovulation and the development of female sexual characteristics and is generally considered a reproductive hormone. In clinical and research settings, the range of estradiol levels is quite wide. In prepubertal children, adolescents, adult women (premenopausal, pregnant, and postmenopausal), and men, physiological estradiol levels range from approximately 1 to 20,000 pg/mL. This range also includes estradiol levels obtained from breast cancer patients treated with aromatase inhibitors, postmenopausal women treated with estrogen, and patients undergoing ovarian stimulation therapy. In patients treated with aromatase inhibitors, this range can extend to a lower limit of <1 pg/mL. Therefore, estradiol assays used in clinical and research laboratories require accuracy and precision over a concentration range of several orders of magnitude. Therefore, it is highly necessary to develop means capable of detecting estradiol concentrations at low levels, such as pg/mL.

One method for determining estradiol levels in blood is immunoassay. However, most current estradiol immunoassays are based on the competitive principle, while the competitive assays inherently preclude measurement of very low analyte concentrations (Simon Rattle et al., Handbook of Immunoassays (4th ed.), 2023, p. 178). Furthermore, estradiol is a low molecular weight substance that cannot accommodate two distinct antibody epitopes to perform a classic sandwich-type immunoassay, which further complicates the detection.

### Contents of the Present Invention

After extensive research and creative work, the present inventors have developed an antibody against estradiol immunocomplex, which is capable of specifically binding to the estradiol immunocomplex, but not specifically binding to the estradiol that constitutes the estradiol immunocomplex, nor specifically binding to the anti-estradiol antibody that constitutes the estradiol immunocomplex. The following invention is thus provided.

One aspect of the present invention relates to an antibody or an antigen-binding fragment thereof against estradiol immunocomplex, wherein the antibody or antigen-binding fragment thereof against estradiol immunocomplex comprises a heavy chain variable region and a light chain variable region, the heavy chain variable region comprises HCDR1, HCDR2, and HCDR3, and the light chain variable region comprises LCDR1, LCDR2, and LCDR3, wherein:
the HCDR1, HCDR2, and HCDR3 comprise amino acid sequences selected from the group consisting of SEQ ID NO: 1, SEQ ID NO: 2, SEQ ID NO: 3, SEQ ID NO: 7, SEQ ID NO: 8, SEQ ID NO: 9, SEQ ID NO: 13, SEQ ID NO: 14, and SEQ ID NO: 15, and the amino acid sequences of HCDR1, HCDR2, and HCDR3 are different from each other; and
the LCDR1, LCDR2, and LCDR3 comprise amino acid sequences selected from the group consisting of SEQ ID NO: 4, SEQ ID NO: 5, SEQ ID NO: 6, SEQ ID NO: 10, SEQ ID NO: 11, SEQ ID NO: 12, SEQ ID NO: 16, SEQ ID NO: 17, and SEQ ID NO: 18, and the amino acid sequences of LCDR1, LCDR2, and LCDR3 are different from each other.

In some embodiments of the present invention, in the antibody or antigen-binding fragment thereof against estradiol immunocomplex:
HCDR1 comprises an amino acid sequence selected from the group consisting of SEQ ID NO: 1, SEQ ID NO: 7, and SEQ ID NO: 13;
HCDR2 comprises an amino acid sequence selected from the group consisting of SEQ ID NO: 2, SEQ ID NO: 8, and SEQ ID NO: 14;
HCDR3 comprises an amino acid sequence selected from the group consisting of SEQ ID NO: 3, SEQ ID NO: 9, and SEQ ID NO: 15;
LCDR1 comprises an amino acid sequence selected from the group consisting of SEQ ID NO: 4, SEQ ID NO: 10, and SEQ ID NO: 16;
LCDR2 comprises an amino acid sequence selected from the group consisting of SEQ ID NO: 5, SEQ ID NO: 11, and SEQ ID NO: 17; and
LCDR3 comprises an amino acid sequence selected from the group consisting of SEQ ID NO: 6, SEQ ID NO: 12, and SEQ ID NO: 18.

In some embodiments of the present invention, in the antibody or antigen-binding fragment thereof against estradiol immunocomplex:
HCDR1 comprises the amino acid sequence as set forth in SEQ ID NO: 1, HCDR2 comprises the amino acid sequence as set forth in SEQ ID NO: 2, HCDR3 comprises the amino acid sequence as set forth in SEQ ID NO: 3, LCDR1 comprises the amino acid sequence as set forth in SEQ ID NO: 4, LCDR2 comprises the amino acid sequence as set forth in SEQ ID NO: 5, and LCDR3 comprises the amino acid sequence as set forth in SEQ ID NO: 6;
HCDR1 comprises the amino acid sequence as set forth in SEQ ID NO: 7, HCDR2 comprises the amino acid sequence as set forth in SEQ ID NO: 8, HCDR3 comprises the amino acid sequence as set forth in SEQ ID NO: 9, LCDR1 comprises the amino acid sequence as set forth in SEQ ID NO: 10, LCDR2 comprises the amino acid sequence as set forth in SEQ ID NO: 11, and LCDR3 comprises the amino acid sequence as set forth in SEQ ID NO: 12; or
HCDR1 comprises the amino acid sequence as set forth in SEQ ID NO: 13, HCDR2 comprises the amino acid sequence as set forth in SEQ ID NO: 14, HCDR3 comprises the amino acid sequence as set forth in SEQ ID NO: 15, LCDR1 comprises the amino acid sequence as set forth in SEQ ID NO: 16, LCDR2 comprises the amino acid sequence as set forth in SEQ ID NO: 17, and LCDR3 comprises the amino acid sequence as set forth in SEQ ID NO: 18.

In some embodiments of the present invention, the antibody or antigen-binding fragment thereof against estradiol immunocomplex, wherein:
the antibody or antigen-binding fragment thereof against estradiol immunocomplex comprises a non-CDR region, and the non-CDR region is derived from a rat antibody, a mouse antibody, a rabbit antibody, or a human antibody;
preferably, the antibody against estradiol immunocomplex is a monoclonal antibody;
preferably, the antibody against estradiol immunocomplex is a murine monoclonal antibody.

In some embodiments of the present invention, in the antibody or antigen-binding fragment thereof against estradiol immunocomplex:
the antibody or antigen-binding fragment thereof against estradiol immunocomplex is selected from the group consisting of Fab, Fab', F(ab')₂, Fd, Fv, dAb, single chain variable fragment (scFv), humanized antibody, or chimeric antibody.

In some embodiments of the present invention, in the antibody or antigen-binding fragment thereof against estradiol immunocomplex:
the K_{D} of the antibody or antigen-binding fragment thereof against estradiol immunocomplex to the estradiol immunocomplex is less than or equal to 10⁻⁸ M, less than or equal to 10⁻⁹ M, or less than or equal to 5x10⁻¹⁰ M.

In some embodiments of the present invention, in the antibody or antigen-binding fragment thereof against estradiol immunocomplex:
the estradiol immunocomplex is a complex formed by binding estradiol to an anti-estradiol antibody;
preferably, the anti-estradiol antibody is the E2 monoclonal antibody with the product number AB-E2-01 from Wuhan Quanjing.

In some embodiments of the present invention, in the antibody or antigen-binding fragment thereof against estradiol immunocomplex:
the antibody or antigen-binding fragment thereof against estradiol immunocomplex is incapable of specifically binding to estradiol alone, and incapable of specifically binding to an anti-estradiol antibody alone;
preferably, the antibody against estradiol immunocomplex is produced by a hybridoma cell line with a deposit number of H-224, H-225, or H-226:
   and the hybridoma cell line is deposited in the All-Russian National Collection of Industrial Microorganisms (VKPM) NRC "Kurchatov Institute" on September 17, 2024.

Another aspect of the present invention relates to an isolated nucleic acid molecule, which encodes the antibody or antigen-binding fragment thereof against estradiol immunocomplex as described in any item of the present invention.

Another aspect of the present invention relates to a recombinant vector, which comprises the isolated nucleic acid molecule of the present invention.

Another aspect of the present invention relates to a host cell, which comprises the isolated nucleic acid molecule of the present invention, or comprises the recombinant vector of the present invention.

Another aspect of the present invention relates to a hybridoma cell line, which has a deposit number of H-224, H-225, or H-226, and the hybridoma cell line is deposited in the All-Russian National Collection of Industrial Microorganisms (VKPM) NRC "Kurchatov Institute" on September 17, 2024.

Another aspect of the present invention relates to a method for preparing the antibody or antigen-binding fragment thereof against estradiol immunocomplex as described in any one of the items of the present invention, comprising culturing the host cell or hybridoma cell of the present invention under suitable conditions, and recovering the antibody or antigen-binding fragment thereof against estradiol immunocomplex from the cell culture.

Another aspect of the present invention relates to a conjugate, which comprises an antibody portion and a conjugation portion, wherein the antibody portion is the antibody or antigen-binding fragment thereof against estradiol immunocomplex as described in any one of the items of the present invention, and the conjugation portion is a detectable label; preferably, the conjugation portion is a radioisotope, a fluorescent substance, a luminescent substance, a colored substance, or an enzyme;
preferably, the enzyme is one or more selected from the group consisting of alkaline phosphatase, horseradish peroxidase, luciferase, glucose oxidase, and β-galactosidase.

Another aspect of the present invention relates to a kit for detecting estradiol, comprising:
the antibody or antigen-binding fragment thereof against estradiol immunocomplex as described in any one of the items of the present invention, or the conjugate of the present invention;
an antibody capable of specifically binding to estradiol; and
optionally, an estradiol standard.

In some embodiments of the present invention, the kit comprises the antibody capable of specifically binding to estradiol; preferably, the antibody capable of specifically binding to estradiol is the E2 monoclonal antibody available from Wuhan Quanjing with the product number AB-E2-01.

In some embodiments of the present invention, in the kit, the antibody capable of specifically binding to estradiol is attached to a solid support.

Preferably, the solid support is a spherical or planar solid support.

Preferably, the solid support is selected from the group consisting of glass slide, polymer beads, magnetic beads, ceramics, silicon wafers, or plastic materials.

Preferably, the antibody is attached to the solid support via biotin-streptavidin, SpyTag/SpyCatcher, receptor-ligand, or OsASI1-BAHOsSUVH6.

In some embodiments of the present invention, in the kit,
the antibody or antigen-binding fragment thereof against estradiol immunocomplex or the conjugate has a concentration of 0.05 to 5 µg/ml, 0.1 to 3 µg/ml, 0.2 to 2 µg/ml, 0.3 to 1 µg/ml, 0.4 to 0.8 µg/ml, or 0.5 µg/ml;
the antibody capable of specifically binding to estradiol has a concentration of 0.05 to 2 µg/ml, 0.1 to 1 µg/ml, 0.1 to 0.8 µg/ml, 0.15 to 0.6 µg/ml, 0.2 to 0.4 µg/ml, or 0.25 mg/ml;
preferably, the antibody or antigen-binding fragment thereof against estradiol immunocomplex or the conjugate is dissolved in a buffer (e.g., Tris-HCl buffer or MES buffer);
preferably, the antibody capable of specifically binding to estradiol is dissolved in a buffer (e.g., Tris-HCl buffer or MES buffer);
preferably, the buffer independently contains NaCl, BSA, Tween-20, and ProClin 300, and one or more selected from the group consisting of NaN₃, MgCl₂, and ZnCl₂.

In some embodiments of the present invention, in the kit, the Tris-HCl buffer contains NaCl, BSA, Tween-20, ProClin 300, and NaN₃; preferably, the Tris-HCl buffer contains 150 mM NaCl, 2% BSA, 0.2% Tween-20, 0.05% ProClin 300, and 0.09% NaN₃.

In some embodiments of the present invention, in the kit, the MES buffer contains NaCl, BSA, Tween-20, ProClin 300, MgCl₂, and ZnCl₂; preferably, the MES buffer contains 0.9% NaCl, 1% BSA, 0.05% Tween-20, 0.048% ProClin 300, 2 mM MgCl₂, and 0.1 mM ZnCl₂.

In some embodiments of the present invention, the kit has a LoQ for detecting estradiol that is less than 10 pg/mL, less than 9 pg/mL, less than 8 pg/mL, less than 7 pg/mL, less than 6 pg/mL, less than 5 pg/mL, less than 4 pg/mL, less than 3 pg/mL, less than 2 pg/mL, less than 1 pg/mL, 1 to 10 pg/mL, 1 to 9 pg/mL, 1 to 8 pg/mL, 1 to 7 pg/mL, 1 to 6 pg/mL, 1 to 5 pg/mL, 1 to 4 pg/mL, or 1 to 3 pg/mL.

Another aspect of the present invention relates to a use of the antibody or antigen-binding fragment thereof against estradiol immunocomplex as described in any one of the items of the present invention, or the conjugate of the present invention, in the manufacture of a reagent or kit for detecting estradiol.

The antibody or antigen-binding fragment thereof against estradiol immunocomplex as described in any one of the items of the present invention or the conjugate of the present invention or the kit of the present invention, for use in detecting estradiol; preferably, the kit is a kit for a sandwich assay for detecting estradiol.

Another aspect of the present invention relates to a method for detecting the level of estradiol in a sample, including a step of applying the antibody or antigen-binding fragment thereof against estradiol immunocomplex as described in any one of the items of the present invention or the conjugate of the present invention to the sample.

In the present invention, unless otherwise specified, scientific and technical terms used herein have the meanings commonly understood by those skilled in the art. Furthermore, cell culture, molecular genetics, nucleic acid chemistry, immunology laboratory procedures as used herein are all routine procedures widely used in the relevant fields. To facilitate a better understanding of the present invention, definitions and explanations of relevant terms are provided below.

As used herein, the term "antibody" refers to an immunoglobulin molecule typically composed of two pairs of polypeptide chains, each pair comprising a "light" (L) chain and a "heavy" (H) chain. Antibody light chains can be classified as kappa (κ) and lambda (λ) light chains. Heavy chains can be classified as µ, δ, γ, α or ε, which further define the antibody isotypes as IgM, IgD, IgG, IgA, and IgE, respectively. Within light and heavy chains, the variable and constant regions are connected by a "J" region of approximately 12 or more amino acids. Heavy chains also contain a "D" region of approximately 3 or more amino acids. Each heavy chain consists of a heavy chain variable region (V_{H}) and a heavy chain constant region (Cu). The heavy chain constant region is composed of three domains: C_{H1}, C_{H2}, and C_{H3}. Each light chain consists of a light chain variable region (V_{L}) and a light chain constant region (C_{L}). The light chain constant region consists of one domain, C_{L}. The constant region of an antibody mediates the binding of the immunoglobulin to host tissues or factors, including various cells of the immune system (e.g., effector cells) and the first component (C1q) of the classical complement system. The V_{H} and V_{L} regions are further subdivided into highly variable regions known as complementarity-determining regions (CDRs), interspersed with more conserved regions known as framework regions (FRs). Each V_{H} and V_{L} region consists of three CDRs and four FRs, arranged from amino-terminus to carboxyl-terminus in the following order: FR1, CDR1, FR2, CDR2, FR3, CDR3, FR4. The variable regions (V_{H} and V_{L}) of each heavy/light chain pair form an antigen-binding site. The CDR sequences of heavy and light chain variable regions are defined by the Kabat numbering system, and the "Kabat numbering system" refers to the EU index as defined by Kabat et al. (see, for example, Kabat, Elvin A., et al. Sequences of Proteins of Immunological Interest. 5th ed., U.S. Dept. of Health and Human Services, Public Health Service, National Institutes of Health, 1991).

The term "antibody" is not limited to any particular method of producing the antibody. For example, it includes, among others, recombinant antibodies, monoclonal antibodies, and polyclonal antibodies. Antibodies can be of different isotypes, e.g., IgG (e.g., IgG1, IgG2, IgG3, or IgG4 subtype), IgA1, IgA2, IgD, IgE, or IgM antibodies.

As used herein, the terms "monoclonal antibody" and "monoclonal antibody" refer to an antibody or antibody fragment from a population of highly homologous antibody molecules, i.e., a population of identical antibody molecules except for any naturally occurring mutations that may occur spontaneously. Monoclonal antibodies have high specificity for a single epitope on an antigen. Polyclonal antibodies, as opposed to monoclonal antibodies, typically contain at least two or more different antibodies, which typically recognize different epitopes on an antigen. Monoclonal antibodies are typically obtained using the hybridoma technique first reported by Kohler et al. (Köhler G, Milstein C. Continuous cultures of fused cells secreting antibody of predefined specificity[J]. Nature, 1975; 256(5517): 495), but can also be obtained using recombinant DNA technology (e.g., see U.S. Patent 4,816,567).

As used herein, the term "humanized antibody" refers to an antibody or antibody fragment obtained by replacing all or part of the CDR regions of a human immunoglobulin (recipient antibody) with the CDR regions of a non-human antibody (donor antibody), wherein the donor antibody can be a non-human (e.g., mouse, rat, or rabbit) antibody with the desired specificity, affinity, or reactivity. In addition, some amino acid residues in the framework region (FR) of the recipient antibody may also be replaced with amino acid residues of the corresponding non-human antibody, or with amino acid residues of other antibodies, to further improve or optimize the performance of the antibody. For more details on humanized antibodies, see, for example, Jones et al., Nature 1986; 321:522-525; Reichmann et al., Nature 1988; 332:323-329; Presta, Curr. Op. Struct. Biol., 1992; 2:593-596; and Clark M. Antibody humanization: a case of the 'Emperor's new clothes'?[J]. Immunol. Today, 2000; 21(8): 397-402.

As used herein, the term "light chain" includes full-length light chains and fragments thereof having sufficient variable region sequence to confer binding specificity. A full-length light chain comprises a variable region domain (VL) and a constant region domain (CL). The variable region domain of the light chain is at the amino terminus of the polypeptide. Light chains include κ and λ, chains.

As used herein, the term "heavy chain" includes full-length heavy chains and fragments thereof having sufficient variable region sequence to confer binding specificity. A full-length heavy chain comprises a variable region domain (VH) and three constant region domains (CH1, CH2, and CH3). The VH domain is at the amino terminus of the polypeptide, and the CH domains are at the carboxyl terminus, with CH3 being closest to the carboxyl terminus. Heavy chains can be of any isotypes, including IgG (including IgG1, IgG2, IgG3, and IgG4 subtypes), IgA (including IgA1 and IgA2 subtypes), IgM, and IgE.

As used herein, the term "antigen-binding fragment" of an antibody refers to a polypeptide comprising a fragment of a full-length antibody that retains the ability to specifically bind to the same antigen bound by the full-length antibody and/or competes with the full-length antibody for specific binding to the antigen, and is also referred to as an "antigen-binding portion". See generally, Fundamental Immunology, Ch. 7 (Paul, W., ed., 2nd edition, Raven Press, N.Y. (1989), which is incorporated herein by reference in its entirety for all purposes. Antigen-binding fragments of antibodies can be produced by recombinant DNA technology or by enzymatic or chemical cleavage of intact antibodies. In some cases, antigen-binding fragments include Fab, Fab', F(ab')₂, Fd, Fv, dAb and complementarity determining region (CDR) fragments, single-chain antibodies (e.g., scFv), chimeric antibodies, diabodies, and polypeptides that contain at least a portion of antibody sufficient to confer specific antigen-binding ability on the polypeptide.

As used herein, the term "Fab fragment" consists of one light chain, CH1 and a variable region of one heavy chain. The heavy chain of a Fab molecule is incapable of forming disulfide bonds with another heavy chain molecule.

As used herein, the term "Fab' fragment" refers to a fragment comprising one light chain and a portion of one heavy chain (which contains VH domain and CH1 domain, as well as a portion of the region between the CH1 and CH2 domains) such that an interchain disulfide bond can form between the two heavy chains of two Fab' fragments to form a F(ab')₂ molecule.

As used herein, the term "F(ab')₂ fragment" refers to a fragment comprising two light chains and two heavy chains containing a portion of the constant region between CH1 and CH2 domains, such that an interchain disulfide bond can form between the two heavy chains. The F(ab')₂ fragment thus consists of two Fab' fragments held together by a disulfide bond between the two heavy chains.

As used herein, the term "Fv region" refers to a fragment comprising variable regions from both heavy and light chains but lacking constant regions.

As used herein, the term "Fd" fragment refers to an antibody fragment consisting of VH and CH1 domain (Ward et al., Nature 341: 544-546 (1989)).

As used herein, the term "dAb" fragment (Ward et al., Nature 341: 544-546 (1989)) consists of a VH domain.

As used herein, the term "isolated" or "being isolated" means that a substance is obtained from its natural state by artificial means. If an "isolated" substance or component occurs in nature, it may be that its natural environment has been changed, or that the substance has been separated from its natural environment, or both. For example, a certain unisolated polynucleotide or polypeptide naturally exists in a living animal, and a highly purified polynucleotide or polypeptide isolated from this natural state is called isolated. The term "isolated" or "being isolated" does not exclude the presence of artificial or synthetic substances, nor does it exclude the presence of other impure substances that do not affect the activity of the substance.

As used herein, the term "vector" refers to a nucleic acid delivery vehicle into which a polynucleotide can be inserted. When a vector can express the protein encoded by the inserted polynucleotide, the vector is called an expression vector. The vector can be introduced into a host cell by transformation, transduction, or transfection, so that the genetic material elements it carries are expressed in the host cell. Vectors are well known to those skilled in the art, including but not limited to: plasmids; phagemids; cosmids; artificial chromosomes, such as yeast artificial chromosomes (YAC), bacterial artificial chromosomes (BAC) or P1-derived artificial chromosomes (PAC); bacteriophages such as λ phage or M13 phage, and animal viruses. Animal viruses that can be used as vectors include, but are not limited to, retroviruses (including lentivirus), adenovirus, adeno-associated virus, herpes virus (e.g., herpes simplex virus), poxvirus, baculovirus, papillomavirus, papovavirus (e.g., SV40). A vector can contain multiple elements for controlling expression, including but not limited to, a promoter sequence, a transcription initiation sequence, an enhancer sequence, a selection element, and a reporter gene. Additionally, the vector may also contain a replication origin.

As used herein, the term "host cell" refers to a cell into which a vector can be introduced, including but not limited to prokaryotic cells such as *Escherichia coli* or *Bacillus subtilis,* fungal cells such as yeast cells *or Aspergillus,* insect cells such as *S2 Drosophila* cells or Sf9, or fibroblasts, CHO cells, COS cells, NSO cells, HeLa cells, GS cells, BHK cells, HEK293 cells or human cells.

As used herein, the term "specific binding" refers to a non-random binding reaction between two molecules, such as the reaction between an antibody and its antigen. In certain embodiments, an antibody that specifically binds to an antigen (or is specific for an antigen) means that the antibody binds to the antigen with an affinity (K_{D}) of less than about 10⁻⁵ M, such as less than about 10⁻⁶ M, 10⁻⁷ M, 10⁻⁸ M, 10⁻⁹ M, or 10⁻¹⁰ M or less.

As used herein, the term "K_{D}" refers to a dissociation equilibrium constant of a specific antibody-antigen interaction and is used to describe the binding affinity between antibody and antigen. The smaller the dissociation equilibrium constant, the tighter the antibody-antigen binding and the higher the affinity between the antibody and the antigen. Typically, an antibody binds to an antigen (e.g., an estradiol immunocomplex) with a dissociation equilibrium constant (K_{D}) of less than about 10⁻⁵ M, such as less than about 10⁻⁶ M, 10⁻⁷ M, 10⁻⁸ M, 10⁻⁹ M, or 10⁻¹⁰ M or less. K_{D} can be determined using methods known to those skilled in the art, such as using a Fortebio molecular interaction instrument.

As used herein, the terms "monoclonal antibody" and "McAb" have the same meaning and are used interchangeably; the terms "polyclonal antibody" and "PcAb" have the same meaning and are used interchangeably; and the terms "polypeptide" and "protein" have the same meaning and are used interchangeably. Furthermore, in the present invention, amino acids are generally represented by single-letter and three-letter abbreviations known in the art. For example, alanine can be represented by A or Ala.

In the present invention, the term LoB (Limit of Blank) refers to a limit of detection for a blank sample, which indicates the highest measurement result that can be obtained using a blank sample under specific probability conditions. LoB indicates that even if the sample does not contain the substance to be tested, the test result may not be zero due to factors such as reagent components and instrumentation. Concentrations below the LoB value can be considered to be zero concentration.

In the present invention, the term LoD (Limit of Detection) refers to a limit of detection, which indicates the lowest concentration that can be detected under certain probability conditions. LoD is used to determine whether an analyte is present in a sample, but the specific concentration thereof cannot be determined. For example, if the LoD is 8 ng/mL and the test result is 6 ng/mL, it means that the analyte is indeed present in the sample, but the concentration is too low to be accurately measured.

In the present invention, the term LoQ (Limit of Quantitation) refers to a limit of quantitation, which indicates the lowest concentration that can be accurately measured. LoQ requires not only that an analyte is detected but also that the result is accurate. For example, if the LoQ is 10 ng/mL and the test result is 12 ng/mL, it means that the concentration of the analyte in the sample is 12 ng/mL.

### Beneficial effects of the present invention

The present invention achieves one or more of the following technical effects:
(1) The antibody of the present invention can specifically bind to an estradiol immunocomplex.
(2) The antibody of the present invention cannot bind to estradiol alone.
(3) The antibody of the present invention cannot bind to an estradiol-specific antibody alone.
(4) The antibody of the present invention has no cross-reactivity or very low cross-reactivity with an estradiol analog (estrone, estriol, testosterone, progesterone, aldosterone, etc.), and is significantly superior to a reagent or kit for competitive detection of estradiol.
(5) The kit prepared from the antibody of the present invention can effectively determine estradiol, and exhibits extremely high sensitivity and specificity.

### Brief Description of the Drawings

Figure 1 shows the absorbance histograms of antibodies E2_112, E2_129, and E2_130 of the present invention after incubation with estradiol-primary antibody immunocomplex, primary antibody, E2-BSA, and BSA, respectively. The results show that the antibodies of the present invention react only with estradiol-primary antibody immunocomplex and have no reactivity with primary antibody or E2 alone.
Figures 2A to 2L show the antibody affinity assay curves, wherein the antibody samples are as follows:

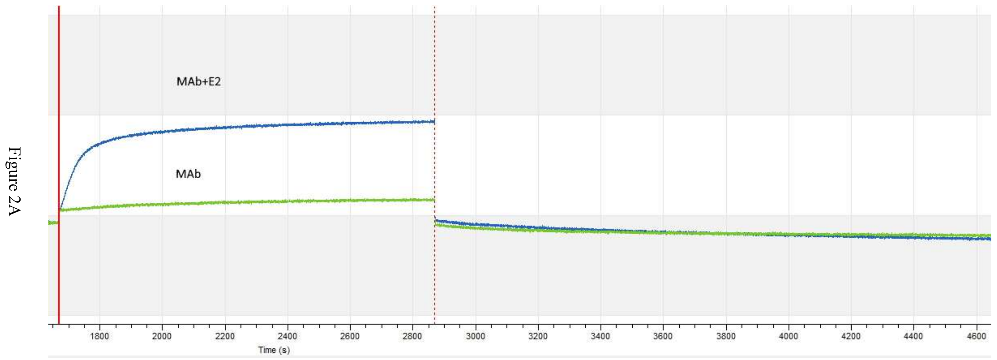

   Figure 2A, E2_112, 5 nM;

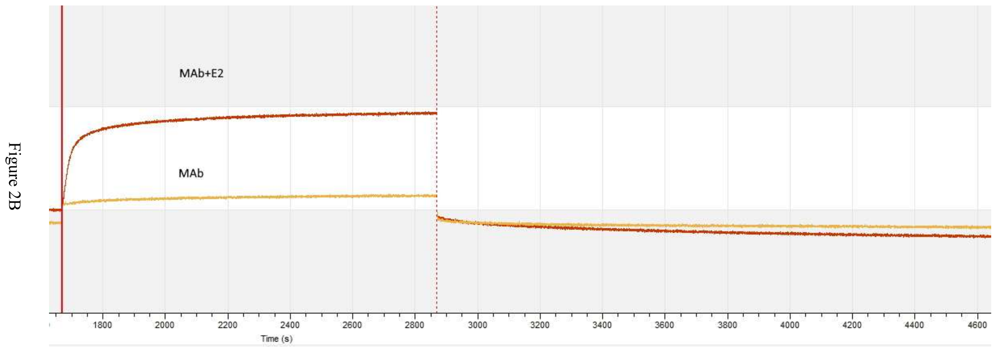

   Figure 2B, E2_112, 10 nM;

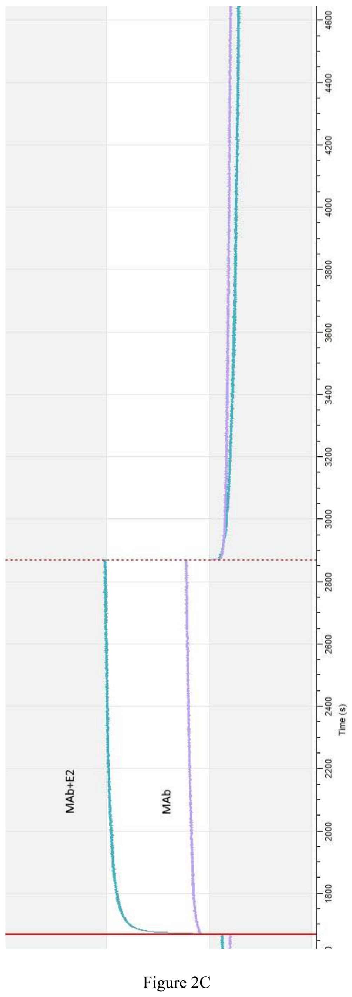

   Figure 2C, E2_112, 50 nMM;

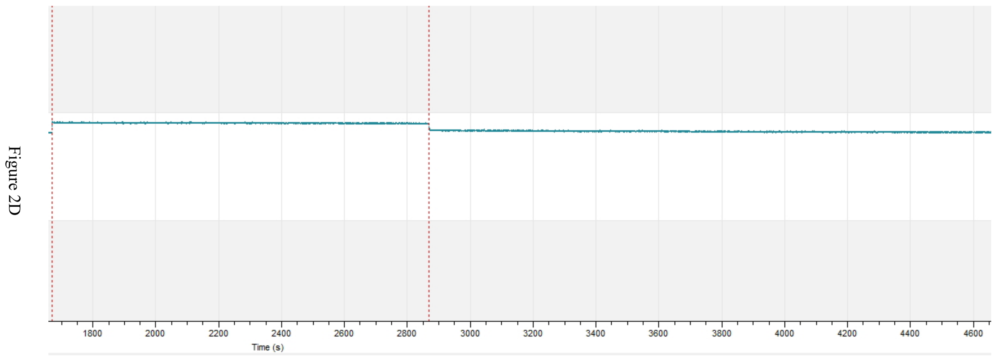

   Figure 2D, E2_112-BSA conjugate;

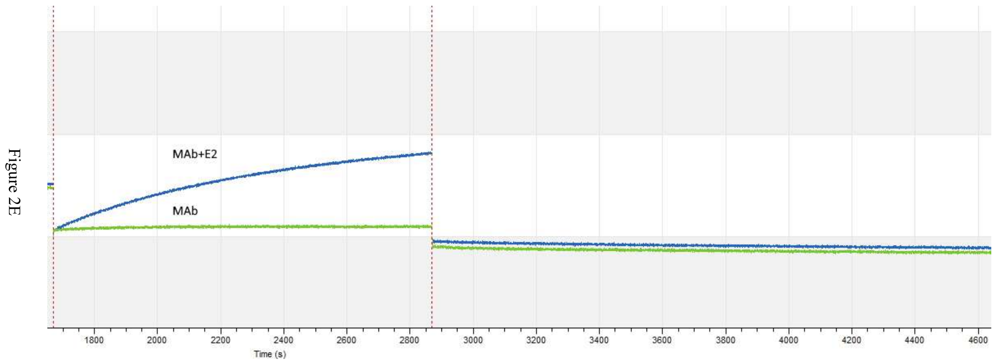

   Figure 2E, E2_129, 5 nM;

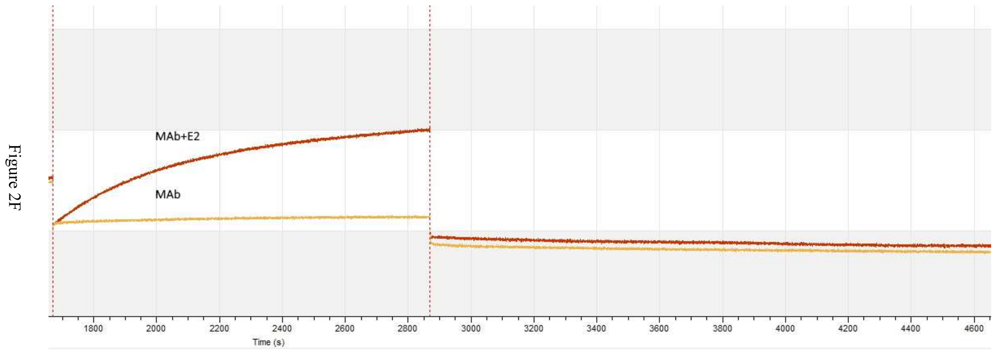

   Figure 2F, E2_129, 10 nM;

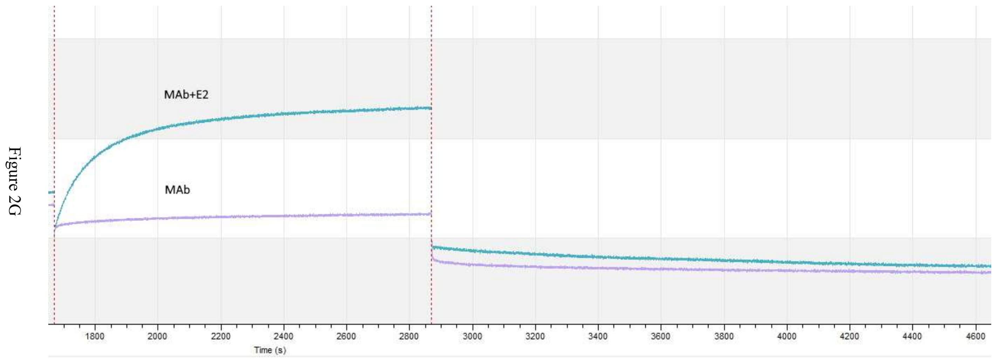

   Figure 2G, E2_129, 50 nM;

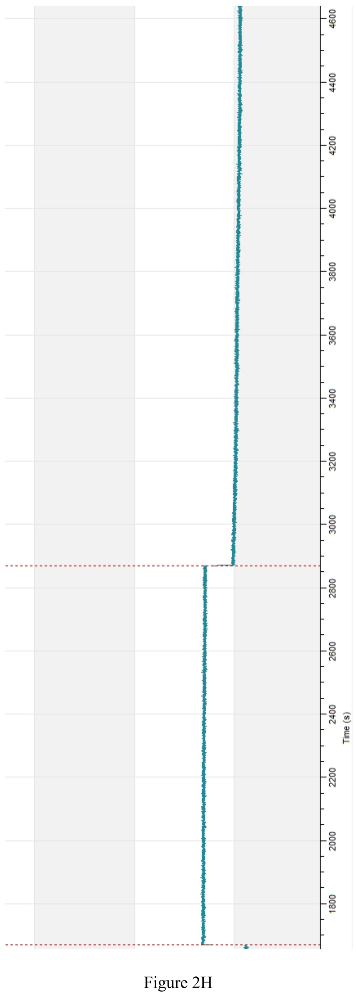

   Figure 2H, E2_129-BSA conjugate;

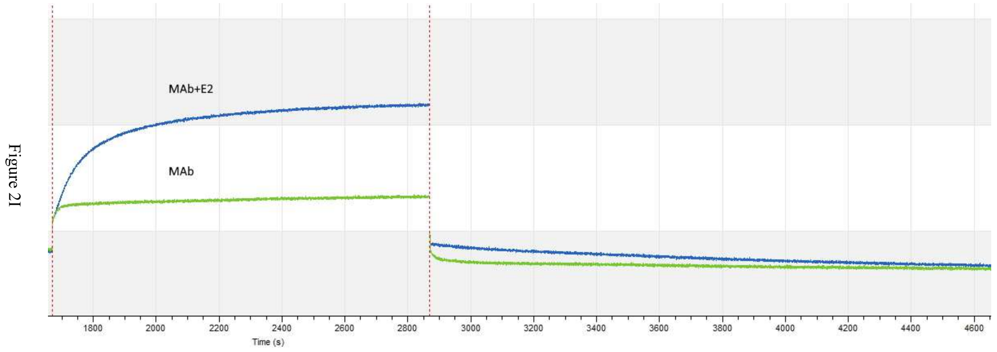

   Figure 2I, E2_130, 5 nM;

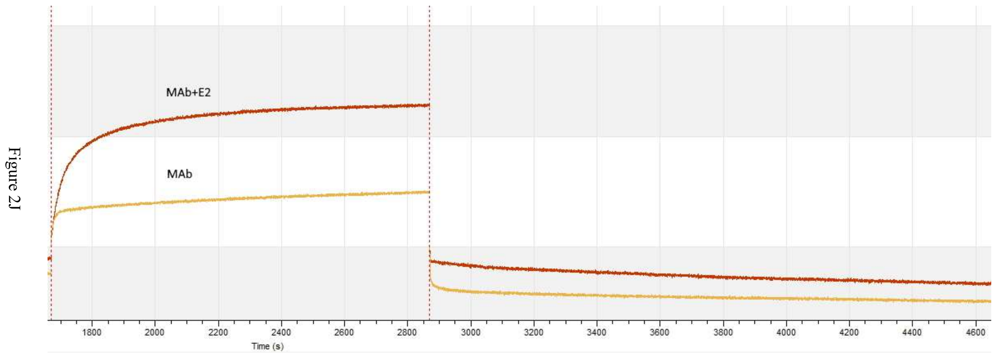

   Figure 2J, E2_130, 10 nM;

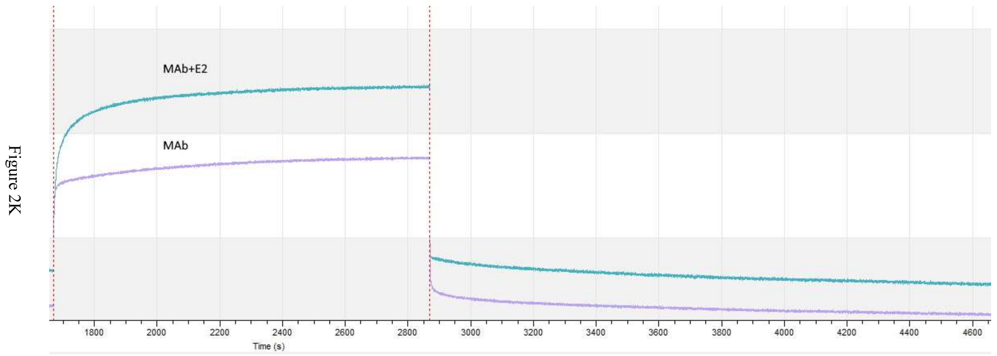

   Figure 2K, E2_130, 50 nM;

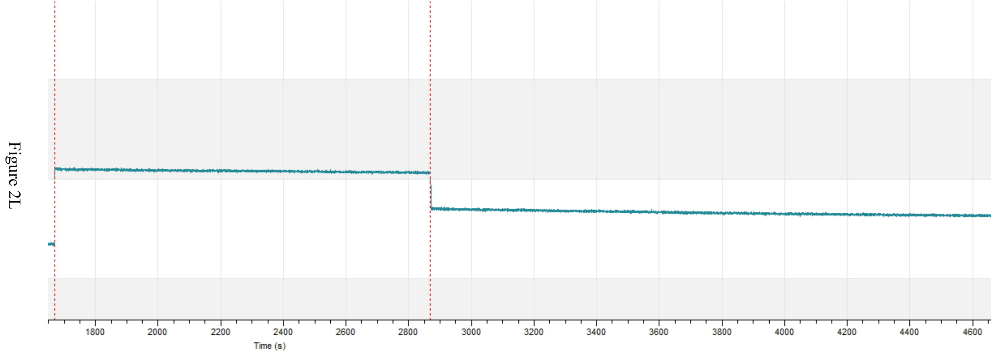

   Figure 2L, E2_130-BSA conjugate.

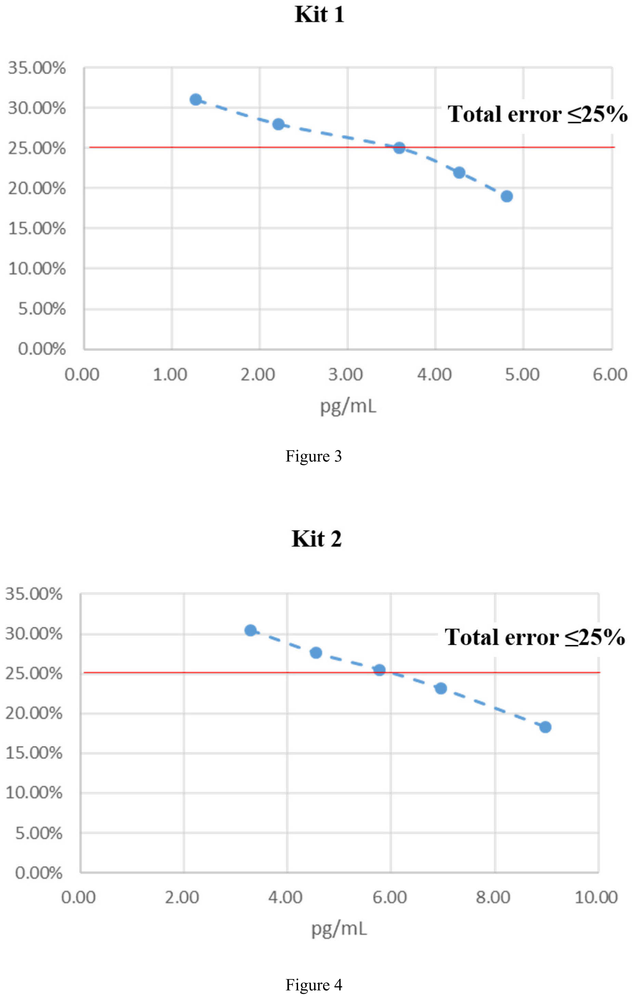

Figure 3 shows the total error of Kit 1 for samples with different concentrations during LoQ establishment.
Figure 4 shows the total error of Kit 2 for samples with different concentrations during LoQ establishment.

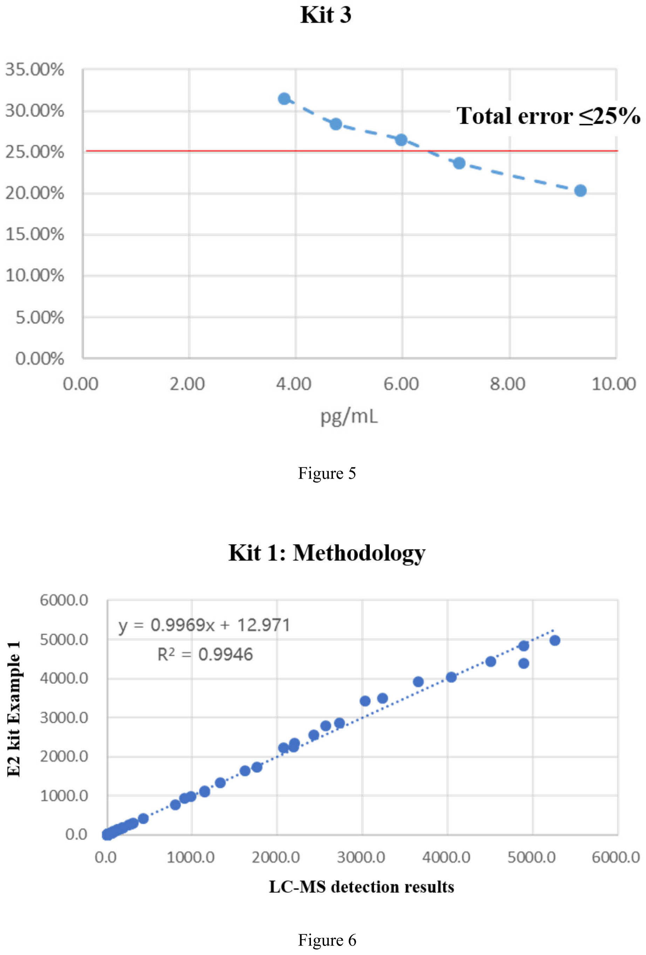

Figure 5 shows the total error of Kit 3 for samples with different concentrations during LoQ establishment.
Figure 6 shows the comparison of Kit 1 with mass spectrometry methodology.

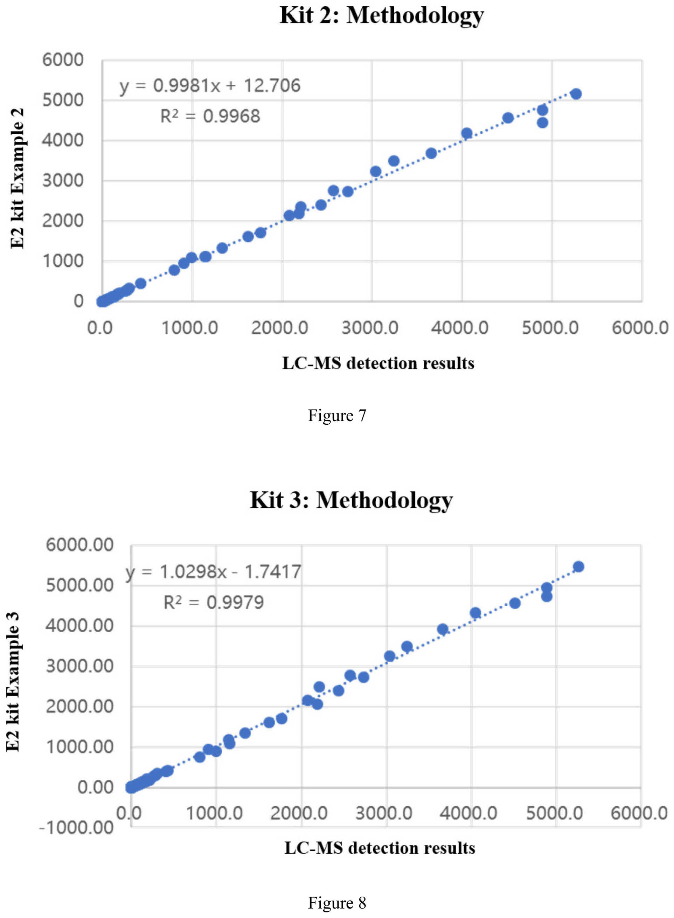

Figure 7 shows the comparison of Kit 2 with mass spectrometry methodology.
Figure 8 shows the comparison of Kit 3 with mass spectrometry methodology.

Deposited Biological Materials:
Hybridoma cell line E2_112, deposited with a deposit number H-224, on September 17, 2024, at the All-Russian National Collection of Industrial Microorganisms (VKPM) NRC «Kurchatov Institute», located at 1st Dorozhny Proezd 1, 117545 Moscow, Russia.
Hybridoma cell line E2_129, deposited with a deposit number H-225, on September 17, 2024, at the All-Russian National Collection of Industrial Microorganisms (VKPM) NRC «Kurchatov Institute», located at 1st Dorozhny Proezd 1, 117545 Moscow, Russia.
Hybridoma cell line E2_130, deposited with a deposit number H-226, on September 17, 2024, at the All-Russian National Collection of Industrial Microorganisms (VKPM) NRC «Kurchatov Institute», located at 1st Dorozhny Proezd 1, 117545 Moscow, Russia.

Partial sequence information related to the present invention is shown in Table A below.

**Table A**

| Name | Sequence | SEQ ID NO: |
|---|---|---|
| E2_112 HCDR1 | SYGVS | 1 |
| E2_112 HCDR2 | TVSPGGSYTDYPDSVKG | 2 |
| E2_112 HCDR3 | GDMITTAMDY | 3 |
| E2_112 LCDR1 | SASSSVSYMY | 4 |
| E2_112 LCDR2 | DTSNLAS | 5 |
| E2_112 LCDR3 | QQWSSYPPT | 6 |
| E2_129 HCDR1 | SYTMS | 7 |
| E2_129 HCDR2 | TISSGGNYTYYPDSVKG | 8 |
| E2_129 HCDR3 | NPHYGSSYDYFDY | 9 |
| E2_129 LCDR1 | RASSSVSYMN | 10 |
| E2_129 LCDR2 | ATSNLAS | 11 |
| E2_129 LCDR3 | QQWSSNPPT | 12 |
| E2_130 HCDR1 | SGYYWN | 13 |
| E2_130 HCDR2 | YISYDGSNNYNPSLKN | 14 |
| E2_130 HCDR3 | DPLYDPFAMDY | 15 |
| E2_130 LCDR1 | HASQNINVWLS | 16 |
| E2_130 LCDR2 | KASNLHT | 17 |
| E2_130 LCDR3 | QQGQSYPYT | 18 |

### Specific Models for Carrying Out the present invention

The following examples describe the present invention in detail. However, those skilled in the art will appreciate that the following examples are intended only to illustrate the present invention and should not be construed as limiting its scope. In the examples, where specific conditions are not specified, the reactions were performed according to conventional conditions or the manufacturer's recommended conditions. Reagents and instruments used without specified manufacturers are all commercially available regular products.

Estradiol (E2) was purchased from Sigma-Aldrich (Cat# E4389).

Anti-estradiol-specific antibody was purchased from Wuhan Quanjing (Cat. No. AB-E2-01, antibody name: E2 monoclonal antibody). E2 monoclonal antibody was also referred to as the primary antibody in the present invention.

HRP-conjugated mouse IgG polyclonal antibody was purchased from Sigma Chemicals, St. Louis, MO. HRP-conjugated mouse IgG polyclonal antibody was also referred to as the secondary antibody in the present invention.

### Example 1: Preparation of hybridoma cell line secreting monoclonal antibody against estradiol immunocomplex

E2 monoclonal antibody was incubated with E2 solution dissolved in PBS at room temperature for 1 hour. The E2 concentration was 100 times the molar concentration of the antibody. Excess estradiol was removed by desalting using a Zeba spin desalting column (Thermo Fisher Scientific) to obtain an estradiol-primary antibody immunocomplex.

The resulting estradiol-primary antibody immunocomplex was used to immunize BALB/c mice according to conventional mouse immunization methods to obtain hybridomas.

The estradiol-primary antibody immunocomplex was used as a pre-adsorbed antigen, and the hybridoma cells producing estradiol-primary antibody immunocomplex-specific antibodies were screened by enzyme-linked immunosorbent assay (ELISA). The specific steps were as follows:
20 ng of estradiol-primary antibody immunocomplex in 0.05 ml PBS was added to each well, and incubated with gentle shaking at room temperature;
after antigen adsorption for 30 minutes, the well was washed twice with PBS containing 0.1% Tween-20 detergent (PBST);
incubation was then performed with 0.05 ml of conditioned medium collected from growing hybridomas for 30 minutes, and washing was carried out twice with PBST;
the mouse antibodies bound to the pre-adsorbed antigen were revealed by incubation with an HRP-conjugated secondary anti-mouse IgG polyclonal antibody (secondary antibody) for 30 minutes, in which 0.05 ml of secondary antibody diluted at 1:1000 in PBST was added to each well;
after incubation with the secondary antibody, washing was carried out six times with PBST, and 0.05 ml of 3,3',5,5'-tetramethylbenzidine (TMB) peroxidase substrate containing 0.03% hydrogen peroxide was added;
after incubation for 10 minutes, the reaction was terminated by adding 0.05 ml of 1 M phosphoric acid, and the absorbance of each well was measured at 450 nm using a Labsystems Multiscan microplate reader (Labsystems, Finland).

The hybridoma cells producing antibodies specific for the estradiol-primary antibody immunocomplex (absorbance at 450 nm greater than 0.5 above background absorbance under the above conditions) were selected for subsequent experiments.

These hybridoma cells were cloned by limiting dilution. Hybridoma clones secreting the target monoclonal antibodies were cultured in Dulbecco's modified Eagle's medium (DMEM) supplemented with 10% fetal bovine serum (HyClone Laboratories, Logan, UT).

Three hybridoma cell lines were obtained, and the antibodies they secreted were designated E2_112, E2_129, and E2_130. These three hybridoma cell lines were deposited in the All-Russian National Collection of Industrial Microorganisms (VKPM) NRC "Kurchatov Institute" on September 17, 2024; their a deposit numbers are H-224, H-225, and H-226, respectively.

### Example 2: Antibody Sequence Analysis

Total RNA was extracted from the murine hybridoma cells obtained in Example 1 using a Quick-RNA Microprep Kit #1050-51 (Zymo Research, USA), reverse-transcribed to generate cDNA and amplified by PCR. The PCR amplification products were sequenced and analyzed to obtain the heavy and light chain variable region sequences of the antibodies. CDRs were annotated using the Kabat numbering system, as shown in Table A above.

### Example 3: Preparation of antibodies

The hybridoma cells producing monoclonal antibodies E2_112, E2_129, and E2_130 were grown in mice, and mouse ascites fluids were used to isolate E2_112, E2_129, and E2_130.

The antibodies (E2_1 12, E2_129, and E2_130) were purified from the ascites fluids using protein A affinity chromatography. The resin was from GE Healthcare Life Sciences (Piscataway, NJ), and purification was performed according to the manufacturer's instructions. The purified monoclonal antibodies were stored at 4°C in 50% ammonium sulfate solution.

### Example 4: Determination of antibody specificity by plate-based indirect ELISA

To determine the specificity of antibodies E2_112, E2_129, and E2_130, 100 ng of either unloaded primary antibody, estradiol-primary antibody immunocomplex, E2-BSA conjugate (β-estradiol 6-(O-carboxymethyl) oxime: BSA, Sigma, E5630), or BSA were adsorbed to the plate surface.

After incubation for 30 minutes, the plate was washed three times with PBST.

A series of dilute solutions of the antibody were prepared in PBST and incubated in the plate wells.

After incubation for 30 minutes, the plate was washed three times with PBST.

HRP-conjugated anti-mouse antibody (Jackson ImmunoResearch, 115-035-003) was added to the plate wells, in which the antibody was diluted at 1:1000 in PBST.

After incubation for 30 minutes, the wells were washed six times with PBST, and 0.05 ml of 3,3',5,5'-tetramethylbenzidine (TMB) peroxidase substrate containing 0.03% hydrogen peroxide was added.

After further incubation for 30 minutes, the reaction was terminated by adding 0.05 ml of 1 M phosphoric acid, and the absorbance of each well was measured at 450 nm using an EnVision microplate reader (Perkin Elmer, USA).

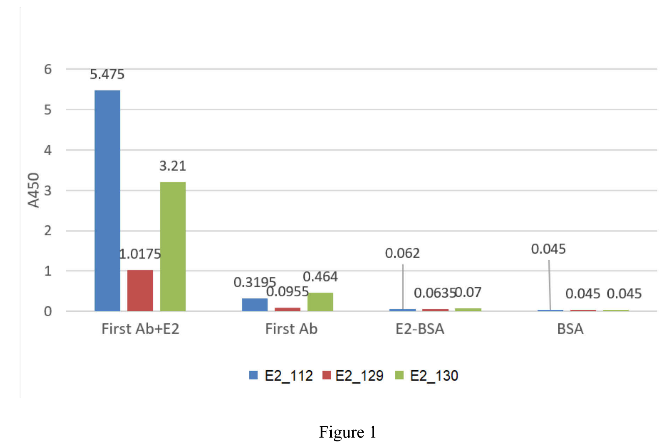

The results are shown in Figure 1. The results showed that the antibodies E2_112, E2_129, and E2_130 reacted exclusively with the estradiol-primary antibody immunocomplex, but not with the primary antibody or E2 alone.

### Example 5: Measurement of antibody affinity

Biolayer interferometry was performed according to the following protocol to determine the affinity of the antibodies for E2 immunocomplex.

Antibody (10 µg/mL) in PBS was bound to a sensor, the sensor was blocked, and then the antigen (primary antibody or estradiol-primary antibody immunocomplex) at concentrations of 5 nM, 10 nM, and 50 nM was reacted with the antibody on the sensor.

The complete experimental protocol was as follows: sensor hydration → antibody binding to sensor → sensor washing → sensor blocking → binding (for each concentration of test antibody) → dissociation → sensor regeneration.

The response of the primary antibody alone was subtracted from the response of the estradiol-primary antibody immunocomplex to generate a response curve for further quantitative analysis. To determine affinity for E2 alone, an E2-BSA conjugate (Sigma E5630) was reacted with the antibody on the sensor. The resulting flat line precluded quantitative analysis, leading to the conclusion that the antibody did not interact with the E2 antigen alone.

The results are shown in Figures 2A to 2L and Table 1.

**Table 1: Affinity data of antibodies**

| Antibody | Ka(1/Ms) | Kd(1/s) | KD(M) |
|---|---|---|---|
| E2_112 | 9.84*10⁵ | 4.24*10⁴ | 4.23*10⁻¹⁰ |
| E2_129 | 8.64*10⁴ | 9.51*10⁻⁶ | 1.10*10⁻¹⁰ |
| E2_130 | 4.78*10⁵ | 4.08*10⁻⁴ | 8.80*10⁻¹⁰ |

The results showed that the antibodies of the present invention had good affinity for the estradiol-primary antibody immunocomplex.

### Example 6: Preparation of kit for estradiol immunoassay

The monoclonal antibodies E2_112, E2_129 and E2_130 were each labeled with alkaline phosphatase (Cat# 03137031103, Roche Custom Biotech) to serve as detection antibodies; E2 monoclonal antibody was labeled with biotin (Cat# PG82075, Thermo) to serve as capture antibody.

The capture antibody was incubated with streptavidin-labeled particles (MyOne T1 Dynabeads Streptavidin PMP Hydrophobic particles 100 mkm, Cat# 35604D, Thermo) to produce capture antibody-labeled magnetic beads. The capture antibody-labeled magnetic beads were used in the assay at conditions that: the capture antibody-labeled magnetic beads were "dissolved" in 50 mM Tris-HCl buffer (pH 7.4) and the concentration of the capture antibody was 0.25 mg/ml, in which 150 mM NaCl, 2% BSA, 0.2% Tween-20, 0.05% ProClin 300, and 0.09% NaN₃ were further contained.

The detection antibody was "dissolved" at a concentration of 0.5 µg/ml in 50 mM MES buffer (pH 6.0) which further contained 0.9% NaCl, 1% BSA, 0.05% Tween-20, 0.048% ProClin 300, 2 mM MgCl₂, and 0.1 mM ZnCl₂.

Estradiol (Sigma Aldrich, E4389) was used as a calibrant in the immunoassay. The estradiol concentrations were 0 pg/mL for Calibrator 1, 500 pg/mL for Calibrator 2, and 4800 pg/mL for Calibrator 3.

### Example 7: Sensitivity detection and methodology comparison

Three kits were prepared according to the previous method. The antibodies used for the magnetic bead reagent and enzyme-labeled reagent are shown in Table 2 below:

**Table 2**

| | Antibodies used in magnetic bead reagent | Antibodies used in enzyme-labeled reagent |
|---|---|---|
| Kit 1 | AB-E2-01 | E2_129 |
| Kit 2 | AB-E2-01 | E2_112 |
| Kit 3 | AB-E2-01 | E2_130 |

Estradiol solutions with different dilution factors were prepared under the conditions of 50 mM Tris-HCl, pH 7.4, 150 mM NaCl and 0.1% Tween-20 (estradiol concentrations were the same as in Example 6).

30 µL of estradiol solution was mixed with 50 µL of magnetic bead suspension and 50 µL of detection monoclonal antibody-phosphatase complex solution. One-step immunoassay was performed on a Mindray CL-6000i fully automated analyzer according to the manufacturer's instructions.

Chemiluminescence was detected using a substrate solution by the Mindray fully automated immunoassay system provided with the analyzer. The active ingredient of the substrate solution was 3-(2-spiroadamantane)-4-methoxy-4-(3-phosphoroxyacyl)-phenyl-1,2-dioxetane (AMPPD).

Chemiluminescence was expressed in relative light units (RLU).

### 7-1: Results of sensitivity test

A series of low-concentration estradiol samples (1 pg/mL, 2 pg/mL, 3 pg/mL, 4 pg/mL, 5 pg/mL, 6 pg/mL, 7 pg/mL, 8 pg/mL, and 9 pg/mL) and a blank sample containing no estradiol were prepared by serial dilution. These samples were tested for four consecutive days using two reagent batches and one instrument, with two replicates per day on each instrument, yielding a total of 32 test results. Following the classical method outlined in the EP17-A2 guideline, the mean and standard deviation of each concentration, as well as the overall standard deviation (SD) and coefficient, were calculated to obtain LoB and LoD. Based on the mean and standard deviation of the low-concentration samples, the deviation of the test value from the reference value and the total error were calculated. The lowest concentration meeting the total error criteria was designated as the LoQ.

The calculated LoB and LoD results were:
Kit 1: LoB = 0.56 pg/mL; LoD = 1.89 pg/mL.
Kit 2: LoB = 1.33 pg/mL; LoD = 3.78 pg/mL.
Kit 3: LoB = 1.54 pg/mL; LoD = 3.89 pg/mL.

The results of the calculated LoQ are shown below (Figures 3 to 5). The LoQ results were all less than 10 pg/mL; in particular, Kit 1 had an LoQ of less than 5 pg/mL. The commercially available E2 kit claims an optimal sensitivity of 10 pg/mL, demonstrating that the kits prepared using the antibodies in this protocol had high sensitivity.

### 7-2: Methodological comparison

120 samples were randomly collected, encompassing samples of varying genders, age groups, and gestational stages. Each sample was divided into two aliquots: one was sent to Mindray's traceability laboratory for mass spectrometry analysis, and the other was analyzed using a Mindray immunoluminescence instrument for luminescence analysis using Kit 1, Kit 2, and Kit 3.

The results are shown in Figures 6 to 8. The results showed that the current E2 detection kit and the LC-MS method had excellent methodological consistency, with correlation coefficients R2 greater than 0.95.

### 7-3: Analog cross-reactivity assay

Kits for competition assay and E2 sandwich assay (Kits 1 to 3 as described above) were developed using E2 monoclonal antibody (primary antibody) to detect analog cross-reactivity. Five analogs (estrogen, estriol, testosterone, progesterone, and aldosterone) at corresponding concentrations were added into E2-free blank serum matrix samples. A total of five samples were prepared, and the concentrations of the samples were detected using the kits for both the competition assay and sandwich assay, respectively. The cross-reactivity rate was calculated as: sample value/analog concentration * 100%. The cross-reactivity rates are shown in Table 3 below.

**Table 3**

| Name | Concentratio n | Cross-reactivity rate | | | |
|---|---|---|---|---|---|
| | | Competitive assay | Kit 1 | Kit 2 | Kit 3 |
| Estrone | 100 ng/mL | 12.39% | 1.10% | 1.10% | 1.10% |
| Estriol | 100 ng/mL | 0.35% | 0.07% | 0.15% | 0.17% |
| Testosterone | 100 ng/mL | 0.02% | 0.01% | 0.01% | 0.01% |
| Progesterone | 100 ng/mL | 0.01% | 0.01% | 0.01% | 0.01% |
| Aldosterone | 100 ng/mL | 0.03% | 0.01% | 0.01% | 0.01% |

The results showed that the kits for E2 sandwich assay of the present invention outperformed the kit for competitive assay in the cross-reactions with four analogs: estrone, estriol, testosterone, and aldosterone. In the cross-reactions with progesterone, the kits for E2 sandwich assay performed on par with that for the competitive assay. These results demonstrated that the kits for sandwich assay of the present invention offered significant advantages over that for the competitive assay in terms of analog interference.

While specific embodiments of the present invention have been described in detail, those skilled in the art will appreciate that, in light of the teachings disclosed herein, various modifications and substitutions may be made to those details, and such variations are within the scope of the present invention. The full scope of the present invention is set forth in the appended claims and any equivalents thereof.

## Claims

1. An antibody or an antigen-binding fragment thereof against estradiol immunocomplex, the antibody or antigen-binding fragment thereof against estradiol immunocomplex comprises a heavy chain variable region and a light chain variable region, the heavy chain variable region comprises HCDR1, HCDR2, and HCDR3, and the light chain variable region comprises LCDR1, LCDR2, and LCDR3, wherein:
HCDR1, HCDR2, and HCDR3 comprise amino acid sequences selected from the group consisting of SEQ ID NO: 1, SEQ ID NO: 2, SEQ ID NO: 3, SEQ ID NO: 7, SEQ ID NO: 8, SEQ ID NO: 9, SEQ ID NO: 13, SEQ ID NO: 14, and SEQ ID NO: 15, and the amino acid sequences of HCDR1, HCDR2, and HCDR3 are different from each other; and
LCDR1, LCDR2, and LCDR3 comprise amino acid sequences selected from the group consisting of SEQ ID NO: 4, SEQ ID NO: 5, SEQ ID NO: 6, SEQ ID NO: 10, SEQ ID NO: 11, SEQ ID NO: 12, SEQ ID NO: 16, SEQ ID NO: 17, SEQ ID NO: 18, and the amino acid sequences of LCDR1, LCDR2, and LCDR3 are different from each other.

2. The antibody or antigen-binding fragment thereof against estradiol immunocomplex according to claim 1, wherein:
HCDR1 comprises an amino acid sequence selected from the group consisting of SEQ ID NO: 1, SEQ ID NO: 7, and SEQ ID NO: 13;
HCDR2 comprises an amino acid sequence selected from the group consisting of SEQ ID NO: 2, SEQ ID NO: 8, and SEQ ID NO: 14;
HCDR3 comprises an amino acid sequence selected from the group consisting of SEQ ID NO: 3, SEQ ID NO: 9, and SEQ ID NO: 15;
LCDR1 comprises an amino acid sequence selected from the group consisting of SEQ ID NO: 4, SEQ ID NO: 10, and SEQ ID NO: 16;
LCDR2 comprises an amino acid sequence selected from the group consisting of SEQ ID NO: 5, SEQ ID NO: 11, and SEQ ID NO: 17; and
LCDR3 comprises an amino acid sequence selected from the group consisting of SEQ ID NO: 6, SEQ ID NO: 12, and SEQ ID NO: 18.

3. The antibody or antigen-binding fragment thereof against estradiol immunocomplex according to any one of claims 1 to 2, wherein:
HCDR1 comprises the amino acid sequence as set forth in SEQ ID NO: 1, HCDR2 comprises the amino acid sequence as set forth in SEQ ID NO: 2, HCDR3 comprises the amino acid sequence as set forth in SEQ ID NO: 3, LCDR1 comprises the amino acid sequence as set forth in SEQ ID NO: 4, LCDR2 comprises the amino acid sequence as set forth in SEQ ID NO: 5, and LCDR3 comprises the amino acid sequence as set forth in SEQ ID NO: 6;
HCDR1 comprises the amino acid sequence as set forth in SEQ ID NO: 7, HCDR2 comprises the amino acid sequence as set forth in SEQ ID NO: 8, HCDR3 comprises the amino acid sequence as set forth in SEQ ID NO: 9, LCDR1 comprises the amino acid sequence as set forth in SEQ ID NO: 10, LCDR2 comprises the amino acid sequence as set forth in SEQ ID NO: 11, and LCDR3 comprises the amino acid sequence as set forth in SEQ ID NO: 12; or
HCDR1 comprises the amino acid sequence as set forth in SEQ ID NO: 13, HCDR2 comprises the amino acid sequence as set forth in SEQ ID NO: 14, HCDR3 comprises the amino acid sequence as set forth in SEQ ID NO: 15, LCDR1 comprises the amino acid sequence as set forth in SEQ ID NO: 16, LCDR2 comprises the amino acid sequence as set forth in SEQ ID NO: 17, and LCDR3 comprises the amino acid sequence as set forth in SEQ ID NO: 18.

4. The antibody or antigen-binding fragment thereof against estradiol immunocomplex according to any one of claims 1 to 3, wherein:
(1) the antibody or antigen-binding fragment thereof against estradiol immunocomplex comprises a non-CDR region, and the non-CDR region is derived from a rat antibody, a mouse antibody, a rabbit antibody, or a human antibody;
(2) the antibody against estradiol immunocomplex is a monoclonal antibody;
(3) the antibody against estradiol immunocomplex is a murine monoclonal antibody;
(4) the antibody or antigen-binding fragment thereof against estradiol immunocomplex is selected from the group consisting of Fab, Fab', F(ab')₂, Fd, Fv, dAb, single chain variable fragment, humanized antibody, or chimeric antibody;
(5) the antibody or antigen-binding fragment thereof against estradiol immunocomplex is capable of binding to the estradiol immunocomplex with a K_{D} of less than or equal to 10⁻⁸ M, less than or equal to 10⁻⁹ M, or less than or equal to 5x10⁻¹⁰ M;
(6) the estradiol immunocomplex is a complex formed by binding estradiol to an anti-estradiol antibody;
(7) the anti-estradiol antibody is an E2 monoclonal antibody with Cat. No. AB-E2-01 from Wuhan Quanjing;
(8) the antibody or antigen-binding fragment thereof against estradiol immunocomplex is incapable of specifically binding to estradiol alone, and incapable of specifically binding to an anti-estradiol antibody alone; and/or,
(9) the antibody against estradiol immunocomplex is capable of being produced by a hybridoma cell line with a deposit number of H-224, H-225, or H-226; and the hybridoma cell line is deposited in the All-Russian National Collection of Industrial Microorganisms (VKPM) NRC "Kurchatov Institute" on September 17, 2024.

5. An isolated nucleic acid molecule, which encodes the antibody or antigen-binding fragment thereof against estradiol immunocomplex according to any one of claims 1 to 4.

6. A recombinant vector, which comprises the isolated nucleic acid molecule according to claim 5.

7. A host cell, which comprises the isolated nucleic acid molecule according to claim 5 or the recombinant vector according to claim 6.

8. A hybridoma cell line, which has a deposit number of H-224, H-225, or H-226, and is deposited in the All-Russian National Collection of Industrial Microorganisms (VKPM) NRC "Kurchatov Institute" on September 17, 2024.

9. A method for preparing the antibody or antigen-binding fragment thereof against estradiol immunocomplex according to any one of claims 1 to 4, comprising culturing the host cell according to claim 7 or the hybridoma cell line according to claim 8 under suitable conditions, and recovering the antibody or antigen-binding fragment thereof against estradiol immunocomplex from the cell culture.

10. A conjugate, which comprises an antibody portion and a conjugation portion, wherein the antibody portion is the antibody or antigen-binding fragment thereof against estradiol immunocomplex according to any one of claims 1 to 4, and the conjugation portion is a detectable label; preferably, the conjugation portion is a radioisotope, a fluorescent substance, a luminescent substance, a colored substance, or an enzyme; preferably, the enzyme is one or more selected from the group consisting of alkaline phosphatase, horseradish peroxidase, luciferase, glucose oxidase, and β-galactosidase.

11. A kit for detecting estradiol, comprising:
the antibody or antigen-binding fragment thereof against estradiol immunocomplex according to any one of claims 1 to 4, or the conjugate according to claim 10,
an antibody capable of specifically binding to estradiol, and
optionally, an estradiol standard;
preferably, the kit is a kit for a sandwich assay for detecting estradiol;
preferably, the antibody capable of specifically binding to estradiol is an E2 monoclonal antibody with Cat. No. AB-E2-01 from Wuhan Quanjing.

12. The kit according to claim 11, wherein the antibody capable of specifically binding to estradiol is attached to a solid support;
preferably, the solid support is a spherical or planar solid support; preferably, the solid support is selected from the group consisting of glass slide, polymer bead, magnetic bead, ceramic, silicon wafer, or plastic material;
preferably, the antibody is attached to the solid support via biotin-streptavidin, SpyTag/SpyCatcher, receptor-ligand, or OsASI1-BAHOsSUVH6.

13. The kit according to any one of claims 11 to 12, wherein:
a concentration of the antibody or antigen-binding fragment thereof against estradiol immunocomplex, or the conjugate according to claim 10, is 0.05 - 5 µg/ml, 0.1 - 3 µg/ml, 0.2 - 2 µg/ml, 0.3 - 1 µg/ml, 0.4 - 0.8 µg/ml, or 0.5 µg/ml;
a concentration of the antibody capable of specifically binding to estradiol is 0.05 - 2 µg/ml, 0.1 - 1 µg/ml, 0.1 - 0.8 µg/ml, 0.15 - 0.6 µg/ml, 0.2 - 0.4 µg/ml, or 0.25 mg/ml;
preferably, the antibody or antigen-binding fragment thereof against estradiol immunocomplex, or the conjugate is dissolved in a buffer (e.g., Tris-HCl buffer or MES buffer);
preferably, the antibody capable of specifically binding to estradiol is dissolved in a buffer (e.g., Tris-HCl buffer or MES buffer);
preferably, the buffer independently comprises NaCl, BSA, Tween-20, and ProClin 300, and one or more selected from the group consisting of NaN₃, MgCl₂, and ZnCl₂.

14. The antibody or antigen-binding fragment thereof against estradiol immunocomplex according to any one of claims 1 to 4 or the conjugate according to claim 10 or the kit according to any one of claims 11 to 13 for use in detecting estradiol; preferably, the kit is a kit for a sandwich assay for detecting estradiol.

15. A method for detecting the level of estradiol in a sample, including a step of applying the antibody or antigen-binding fragment thereof against estradiol immunocomplex according to any one of claims 1 to 4 or the conjugate according to claim 10 to the sample.
